# EUROPEAN PATENT APPLICATION

(11) **EP 4 851 291 A1**
(43) Date of publication of application: **09.12.2026**
(21) Application number: 25748102.8
(22) Date of filing: 24.01.2025
(51) Int. Cl.: C12N 15/115, A61K 31/7088, A61K 47/59, A61K 48/00, A61P 43/00

(54) **CONJUGATE OF APTAMER AND POLYETHYLOXAZOLINE**

(30) Priority: 30.01.2024 JP 2024012285
(71) Applicant: RIBOMIC INC., Minato-ku Tokyo 108-0071 (JP)
(72) Inventor: YAMASHITA, Kohei, Tokyo 108-0071 (JP); ADACHI, Tatsuo, Tokyo 108-0071 (JP)
(74) Representative: Weickmann & Weickmann PartmbB
(86) International application number: PCT/JP2025/002178
(87) International publication number: WO 2025/164520

(57) **Abstract**

A method for enhancing the blood stability of an aptamer, and a conjugate of an aptamer whose stability has been enhanced by the method are provided. The above-mentioned problem is solved by providing a conjugate of an aptamer and polyethyloxazoline, or a salt thereof.

## Description

### [Technical Field]

The present invention relates to a conjugate of an aptamer and polyethyloxazoline, or a salt thereof, and a composition containing the same. The present invention also relates to a method for producing an aptamer having an extended blood half-life, and a method for stabilizing an aptamer.

### [Background Art]

In recent years, application of RNA aptamers to therapeutic agents, diagnostic agents, and reagents is attracting attention, and several RNA aptamers have entered a clinical stage or a practical use stage. In December 2004, Macugen, which is the world's first RNA aptamer drug, was approved in the United States as a therapeutic agent for age-related macular degeneration. An RNA aptamer is an RNA that specifically binds to a target substance such as a protein, and can be produced using the SELEX method (Systematic Evolution of Ligands by Exponential Enrichment) (see Patent Literatures 1 to 3). The SELEX method is a method of selecting RNA that specifically binds to a target substance, from a pool of RNAs having about 10¹⁴ different nucleotide sequences. The RNA used has a structure in which a random sequence of about 40 residues is sandwiched between primer sequences. This RNA pool is associated with a target substance, and only the RNA bound to the target substance is recovered using a filter or the like. The recovered RNA is amplified by RT-PCR, and this is used as a template for the next round. By repeating this operation about 10 times, an RNA aptamer that specifically binds to the target substance can sometimes be obtained.

As described above, while RNA aptamers are promising as therapeutic agents, diagnostic agents, and reagents, they are characteristically easily excreted from the body due to their low molecular weight. Therefore, particularly when a RNA aptamer is used as a therapeutic agent, it is necessary to extend the blood half-life thereof.

To date, as a technology for extending the blood half-life of a substance used as a pharmaceutical, a technology using polyethylene glycol (hereinafter referred to as "PEG") has been reported. For example, Non Patent Literature 1 describes that by bonding PEG to an RNA aptamer, the pharmacokinetic properties of the RNA aptamer are remarkably improved (the blood half-life is extended).

### [Citation List]

### [Patent Literature]

[Patent Literature 1]
   WO 91/19813
[Patent Literature 2]
   WO 94/08050
[Patent Literature 3]
   WO 95/07364

### [Non Patent Literature]

[Non Patent Literature 1]
Shin Miyakawa, Masatoshi Fujiwara, Michihisa Nishiyama, Development of Molecularly Targeted Drugs Using RNA Aptamers, Drug Delivery System, 2008, Vol. 23, No. 5, pp. 534-543.

### [Summary of Invention]

### [Technical Problem]

As described above, although PEG is a substance that exhibits an excellent effect in extending blood half-life in the body, it may cause an allergic reaction, and there was room for improvement in this respect.

Accordingly, an object of the present invention is to provide a method for enhancing the blood stability of an aptamer without using PEG, and a conjugate of an aptamer whose blood stability has been enhanced by the method.

### [Solution to Problem]

The present inventors have conducted intensive studies in an attempt to solve the above-mentioned problems and found that the blood half-life is extended by bonding polyethyloxazoline (also referred to as "PEOZ") to an aptamer. The present inventors have also found that, in the above-mentioned embodiment, the blood half-life is further extended by using branched polyethyloxazoline in which linear polyethyloxazoline is bonded to a branched linker (hereinafter also simply referred to as "branched polyethyloxazoline"), and a blood half-life equivalent to or superior to the case where PEG is added is exhibited, which resulted in the completion of the present invention.

That is, one aspect of the present invention relates to the following.
[1] A conjugate of an aptamer and polyethyloxazoline, or a salt thereof.
[2] The conjugate or a salt thereof according to [1], wherein the aforementioned polyethyloxazoline forms a branched chain.
[3] The conjugate or a salt thereof according to [1] or [2], wherein the aforementioned polyethyloxazoline is bonded to the 5' end of the aforementioned aptamer.
[4] The conjugate or a salt thereof according to any of [1] to [3], wherein the aforementioned polyethyloxazoline is two in number and the two polyethyloxazolines form a branched chain via a linker.
[5] The conjugate or a salt thereof according to any of [2] to [4], wherein each molecular weight of the branched chains of the aforementioned polyethyloxazoline is 20 to 60 kDa.
[6] The conjugate or a salt thereof according to [4], wherein the aforementioned polyethyloxazoline is bonded to the aforementioned linker by a dipolar cycloaddition reaction.
[7] The conjugate or a salt thereof according to [6], wherein the aforementioned dipolar cycloaddition reaction is a dipolar cycloaddition reaction between an azide on the side of the aforementioned polyethyloxazoline and a dibenzocyclooctyne on the side of the aforementioned linker.
[8] The conjugate or a salt thereof according to [1], wherein the aforementioned polyethyloxazoline is bonded to the 5' end of the aforementioned aptamer via a linker.
[9] The conjugate or a salt thereof according to [8], wherein the structure of the aforementioned polyethyloxazoline-linker moiety is the following: 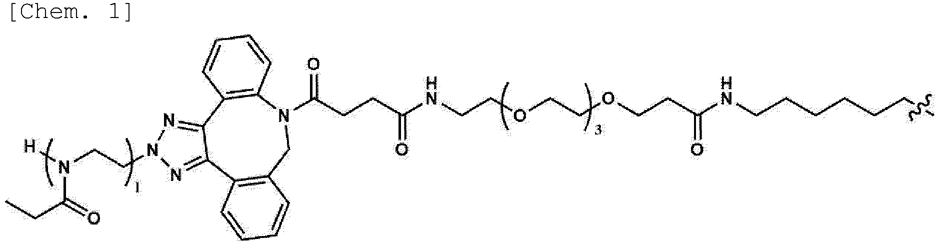 wherein l indicates a degree of polymerization, and l is 200 to 600.
[10] The conjugate or a salt thereof according to [4], wherein the structure of the aforementioned polyethyloxazoline-linker moiety is the following: 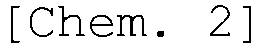 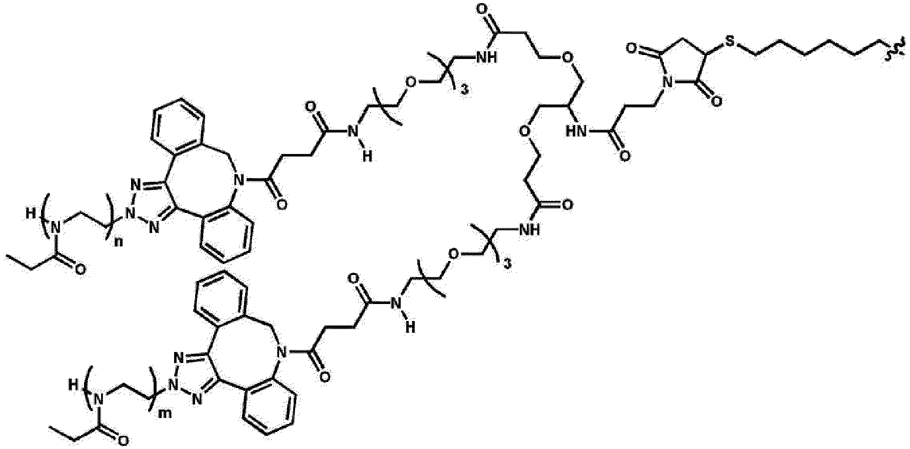 wherein m and n each indicate a degree of polymerization, m is 200 to 600, and n is 200 to 600.
[11] The conjugate or a salt thereof according to any of [1] to [10], wherein the aforementioned aptamer is an anti-FGF2 aptamer or an anti-autotaxin aptamer.
[12] The conjugate or a salt thereof according to any of [1] to [9], wherein the aforementioned aptamer is an aptamer consisting of a nucleotide sequence represented by the following formula (1):

   tC(M)U(M)G(M)A(M)G(M)G(M)gA(M)AsA(M)C(F)A(M)ggU(F)U(F)U(F)U(F)G (M)C(F)U(M)cC(M)U(M)cG(M)G(M)A(M)_idT (1)

   (uppercase letters indicate RNA, lowercase letters indicate DNA, parentheses in nucleotides indicate modification at the 2' position of ribose, F indicates a fluorine atom, and M indicates an O-methyl group; idT at the sequence end indicates modification by inverted-dT, and s in the sequence indicates that the phosphate group linking nucleotides to each other has been phosphorothioated.)
[13] A composition comprising the conjugate or a salt thereof according to any of [1] to [12].
[14] A method for producing an aptamer having an extended blood half-life, comprising a step of bonding polyethyloxazoline to an aptamer.
[15] The production method according to [14], wherein the aforementioned polyethyloxazoline forms a branched chain.
[16] The production method according to [14] or [15], wherein the aforementioned polyethyloxazoline is bonded to the 5' end of the aforementioned aptamer.
[17] The production method according to any of [14] to [16], wherein the aforementioned polyethyloxazoline branched via a linker is bonded to the aforementioned aptamer.
[18] The production method according to any of [15] to [17], wherein the aforementioned polyethyloxazoline in which each molecular weight of the branched chains is 20 to 60 kDa is bonded to the aforementioned aptamer.
[19] The production method according to [17] or [18], wherein the aforementioned polyethyloxazoline and the linker are bonded by a dipolar cycloaddition reaction.
[20] The production method according to [19], wherein the aforementioned polyethyloxazoline and the linker are bonded by a dipolar cycloaddition reaction between an azide on the side of the aforementioned polyethyloxazoline and a dibenzocyclooctyne on the side of the aforementioned linker.
[21] The production method according to any of [14] to [20], wherein the aforementioned polyethyloxazoline branched via a linker is bonded to the aforementioned aptamer by a Michael addition reaction between a maleimide group on the aforementioned linker side and a thiol group on the aforementioned aptamer side.
[22] The production method according to any of [14] to [21], wherein the aforementioned polyethyloxazoline is bonded to the 5' end of the aforementioned aptamer via a linker.
[23] The production method according to [22], wherein the structure of the aforementioned polyethyloxazoline-linker moiety is the following: 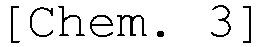 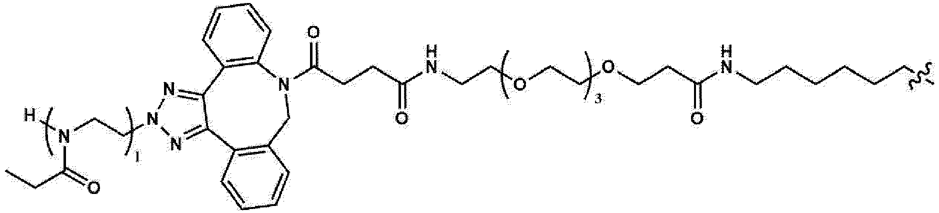 wherein l indicates a degree of polymerization, and l is 200 to 600.
[24] The production method according to [17], wherein the structure of the aforementioned polyethyloxazoline-linker moiety is of the following structural formula: 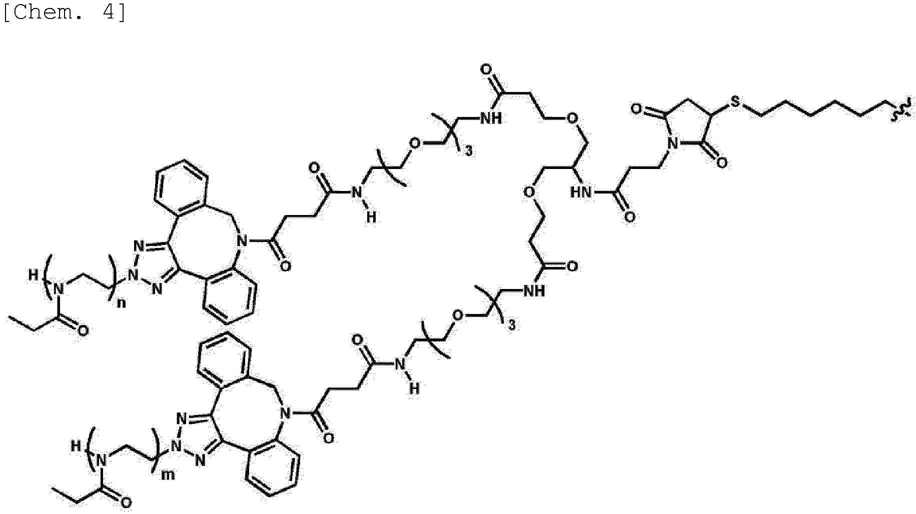 wherein m and n each indicate a degree of polymerization, m is 200 to 600, and n is 200 to 600.
[25] A method for stabilizing an aptamer, comprising bonding polyethyloxazoline to an aptamer.

### [Advantageous Effects of Invention]

According to the present invention, it is possible to provide a conjugate of an aptamer whose blood stability has been enhanced without using PEG. Therefore, the present invention can avoid the problem of an allergic reaction by PEG, and is useful in various treatments using the aptamer.

### [Brief Description of Drawings]

[Fig. 1]

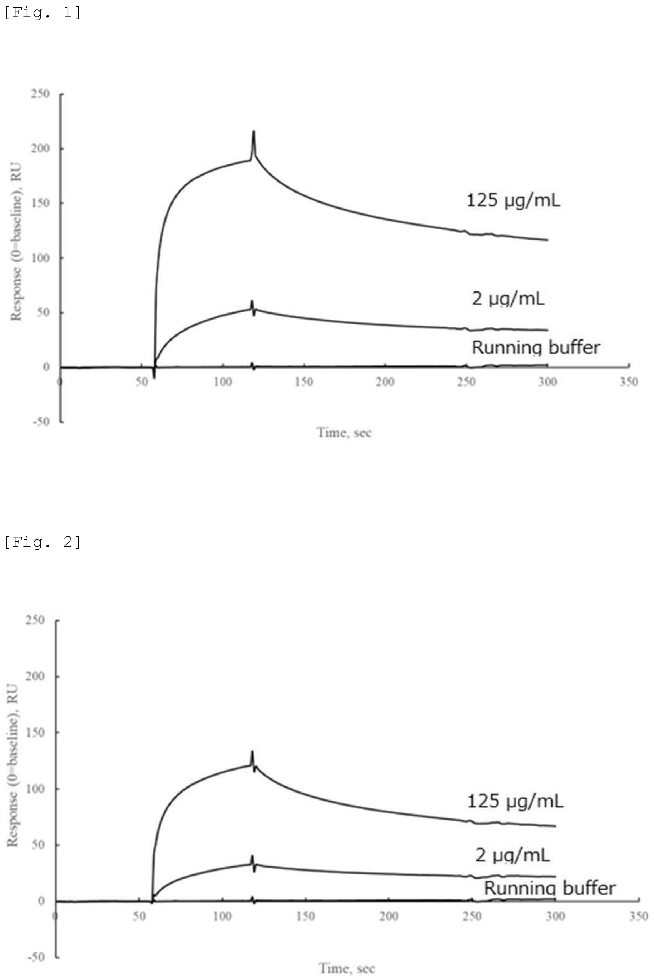

   Fig. 1 is a diagram showing a sensorgram indicating binding between a branched PEOZ (50 kDax2)-added anti-FGF2 aptamer and human FGF2.
[Fig. 2]
   Fig. 2 is a diagram showing a sensorgram indicating binding between a PEG-added anti-FGF2 aptamer and human FGF2.
[Fig. 3]

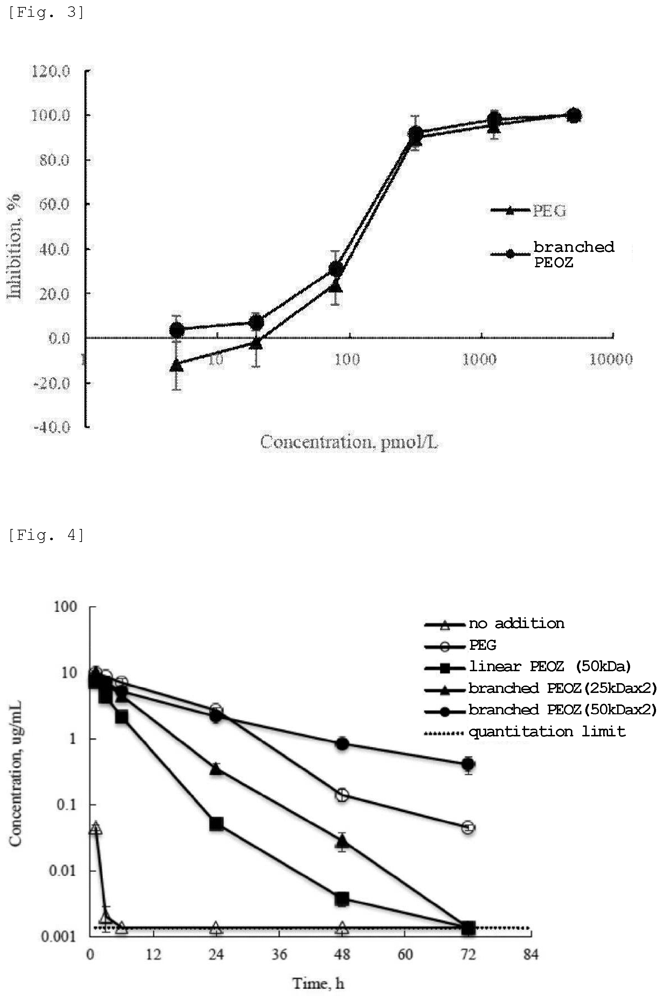

   Fig. 3 is a diagram showing the inhibitory effect on ERK phosphorylation in cells after FGF2 stimulation by addition of a branched PEOZ (50 kDax2)-added anti-FGF2 aptamer and a PEG-added anti-FGF2 aptamer.
[Fig. 4]
   Fig. 4 is a diagram showing the results of blood retention property evaluation of a branched PEOZ (25 kDax2, 50 kDax2), a linear PEOZ (50 kDa), and a PEG-added anti-FGF2 aptamer, and a non-added anti-FGF2 aptamer.
[Fig. 5]

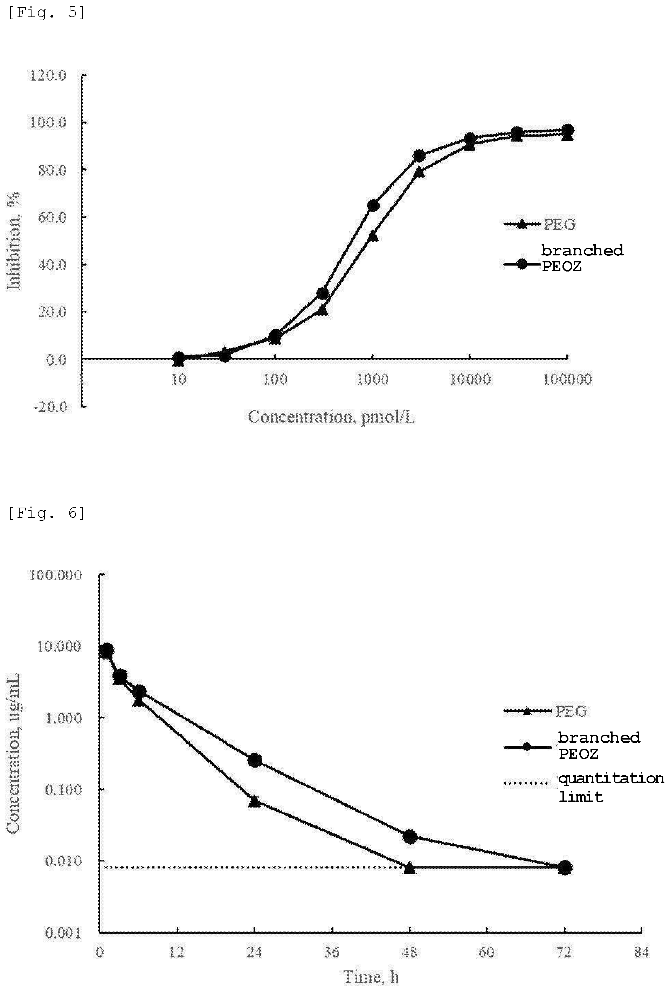

   Fig. 5 is a diagram showing the results of enzyme inhibitory activity evaluation of a branched PEOZ (50 kDax2)-added anti-autotaxin aptamer and a PEG-added anti-autotaxin aptamer.
[Fig. 6]
   Fig. 6 is a diagram showing the results of blood retention property evaluation of a branched PEOZ (50 kDax2)-added anti-autotaxin aptamer and a PEG-added anti-autotaxin aptamer.

### [Description of Embodiments]

In the following, an example of an embodiment of the present invention is described in detail, but the present invention is not limited thereto.

### (1. Definition)

In the present specification, "aptamer" refers to a nucleic acid molecule having a binding activity to a particular target molecule. The aptamer can inhibit the activity of a particular target molecule by binding to the target molecule.

In the present specification, "polyethyloxazoline" has the meaning generally used in the art, and is also denoted as poly(2-ethyl-2-oxazoline).

In the present specification, "conjugate" means a complex composed of two or more substances. In the present invention, the conjugate is composed of at least an aptamer and polyethyloxazoline.

In the present specification, "salt of a conjugate" means a conjugate (complex) existing in the form of a neutralized salt. In the present invention, the salt of a conjugate is a salt of a conjugate composed of at least an aptamer and polyethyloxazoline. In one embodiment of the present invention, the salt of a conjugate may be a pharmaceutically acceptable salt of a conjugate. In the present specification, "pharmaceutically acceptable" means a salt medically suitable for use in contact with tissues of humans or other mammals, without excessive toxicity, irritation, allergic reaction, and the like. Examples of the pharmaceutically acceptable salt include inorganic acid salts such as hydrochloride, hydrobromide, nitrate, sulfate, phosphate, and the like; organic acid salts such as acetate, oxalate, maleate, fumarate, citrate, benzoate, methanesulfonate, and the like; alkali metal salts such as sodium salt, potassium salt, and the like; alkaline earth metal salts such as magnesium salt, calcium salt, and the like; aluminum salt; zinc salt; ammonium salt, tetramethylammonium salt; and addition salts with morpholine, piperidine, lysine, glycine, phenylalanine, aspartic acid, glutamic acid, and the like.

In the present specification, "linker" means a connecting moiety that connects two or more substances. In the present invention, the linker connects an aptamer and polyethyloxazoline.

In the present specification, "anti-FGF2 aptamer" means an aptamer that has binding activity to FGF2 and can inhibit the binding between FGF2 and FGF receptor. That is, the anti-FGF2 aptamer has an inhibitory activity against FGF2.

FGF2 is a protein strongly expressed during early development, differentiation, proliferation, and regeneration, and is, for example, a protein having an amino acid sequence represented by Accession code EAX05222 or NP001997. FGF2 is sometimes called bFGF (basic FGF), FGFB, or HBGF-2.

The inhibitory activity against FGF2 means an inhibitory ability against any activity that FGF2 has. For example, FGF2 acts on FGF receptor-expressing cells to activate signal transduction and induce production of various cell growth factors and receptors thereof. Therefore, the inhibitory activity against FGF2 may be an activity to inhibit intracellular signal transduction via FGF receptor. In addition, since the expression of these various cell growth factors and receptors thereof consequently leads to enhancement of cell proliferation activity and migration activity, the inhibitory activity against FGF2 means inhibition of those activities.

In the present specification, "anti-autotaxin aptamer" means an aptamer having binding activity to autotaxin and capable of inhibiting the enzymatic activity of autotaxin. That is, the anti-autotaxin aptamer has inhibitory activity against autotaxin.

Autotaxin is a glycoprotein present in the blood, and is an enzyme that degrades lysophosphatidylcholine (LPC) into lysophosphatidic acid (LPA) and choline.

The inhibitory activity against autotaxin means an inhibitory ability against any activity that the autotaxin has. While autotaxin has a phosphodiesterase activity to cleave phosphodiester bond by hydrolysis, such activity is inhibited. An acceptable substrate for enzyme activity is not limited to a phosphodiester bond-containing substance (e.g., ATP and the like) present in vivo, and includes a substrate wherein a compound containing same is added with a chromogenic substance or a fluorescent substance. The chromogenic substance and fluorescent substance are known to those of ordinary skill in the art. Also, autotaxin has a lysophospholipase D activity. By this activity, lysophosphatidic acid (LPA) is mainly produced by cleaving the bond on the side of the phosphodiester of a lysophospholipid opposite to the glycerol backbone. Inhibition of autotaxin activity also includes suppression of such production.

In the present specification, "blood half-life" has the meaning generally used in the art, and means the time until the blood concentration of a substance or drug component in a living body decreases to half. The blood half-life can be evaluated, for example, by the blood retention shown in the Examples. In the present invention, the blood half-life is extended by bonding the aptamer to polyethyloxazoline. The extension of the blood half-life can also be rephrased as an increase in blood retention, an increase in blood stability, and the like.

### (2. Conjugate of aptamer and polyethyloxazoline)

In one embodiment of the present invention, a conjugate of an aptamer and polyethyloxazoline (hereinafter also referred to as "the present conjugate") or a salt thereof is provided. In the present conjugate, the blood half-life is extended due to bonding with polyethyloxazoline. In addition, since PEG is not used, the problem of allergic reactions caused by PEG can be avoided.

In the present conjugate, polyethyloxazoline may be bonded to either the 5' end or the 3' end of the aptamer.

In the present conjugate, polyethyloxazoline may be directly bonded to the aptamer, or may be bonded to the aptamer via a linker. Preferably, it is bonded via a linker.

The linker is not particularly limited, and examples thereof include alkyl chain, unsaturated fatty acid chain, cholesterol, polyethylene glycol, polypropylene glycol, polysaccharide, polypeptide, polypeptoid, and the like.

The polyethyloxazoline can be in the form of a linear chain or a branched chain. In one embodiment of the present invention, the present conjugate may be a conjugate in which a linear polyethyloxazoline is bonded to an aptamer via a branched linker (also referred to as a "branched polyethyloxazoline-added aptamer"). In another embodiment of the present invention, the present conjugate may be a conjugate in which a linear polyethyloxazoline is bonded to an aptamer via an unbranched linker (also referred to as a "linear polyethyloxazoline-added aptamer"). Since the blood half-life is further extended by addition to the aptamer, the embodiment is preferably of a branched type.

The molecular weight of the polyethyloxazoline is preferably 20 to 60 kDa. In the case of the branched polyethyloxazoline-added aptamer, each molecular weight of the branched chain of the polyethyloxazoline is preferably 20 to 60 kDa.

In the case where polyethyloxazoline is bonded to the aptamer via a linker, the bonding mode between polyethyloxazoline and the linker is not particularly limited, and examples thereof include binding by a dipolar cycloaddition reaction (also referred to as "dipolar cyclization reaction"), transesterification reaction, Michael addition reaction, SN2 reaction, dehydration condensation reaction, and the like.

When bonding is performed by a dipolar cycloaddition reaction, the binding is preferably performed by a dipolar cycloaddition reaction between an azide on the polyethyloxazoline side and a dibenzocyclooctyne on the linker side.

In the case where polyethyloxazoline is bonded to the aptamer via a linker, the structure of the polyethyloxazoline-linker moiety is preferably the following structure.

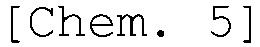

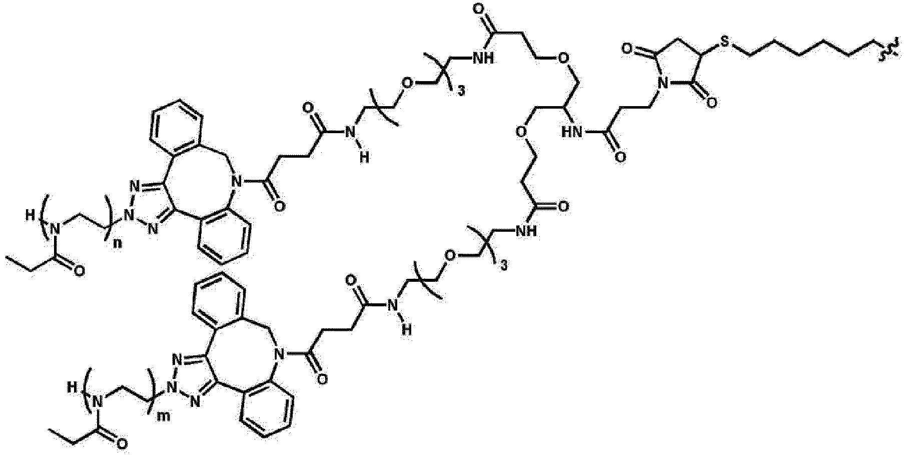

In the above-mentioned structure, m and n each indicate a degree of polymerization. m and n are preferably 200 to 600, more preferably 220 to 550, further preferably 250 to 500.

The aptamer is preferably an anti-FGF2 aptamer or an anti-autotaxin aptamer. The anti-FGF2 aptamer is not particularly limited, and examples thereof include aptamers described in WO 2015/147017, WO 2011/099576, and the like. The anti-autotaxin aptamer is not particularly limited, and examples thereof include aptamers described in WO 2015/163458, WO 2015/147290, and the like. The anti-autotaxin aptamer is preferably a modified variant of the aptamer consisting of the nucleotide sequence represented by SEQ ID NO: 1 shown below (that is, an aptamer consisting of the nucleotide sequence represented by the formula (1)).
SEQ ID NO: 1: 5'-tCUGAGGgAAACAggUUUUGCUcCUcGGA-3' (uppercase letters indicate RNA, lowercase letters indicate DNA.) In SEQ ID NO: 1 of the Sequence Listing, "RNA" is selected as the "molecular type" for convenience.
The formula (1):

tC(M)U(M)G(M)A(M)G(M)G(M)gA(M)AsA(M)C(F)A(M)ggU(F)U(F)U(F)U(F)G (M)C(F)U(M)cC(M)U(M)cG(M)G(M)A(M)_idT

(uppercase letters indicate RNA, lowercase letters indicate DNA, parentheses in nucleotides indicate modification at the 2' position of ribose, F indicates a fluorine atom, and M indicates an O-methyl group; idT at the sequence end indicates modification by inverted-dT, and s in the sequence indicates that the phosphate group linking nucleotides to each other has been phosphorothioated, C and c indicate cytosine, U indicates uracil, G and g indicate guanine, A indicates alanine, and t indicates thymine, respectively).

The aptamer may be an RNA aptamer or a DNA aptamer. RNA aptamers, which have particularly high production costs, become more useful because blood stability is imparted by the present invention.

### (3. Composition)

In one embodiment of the present invention, a composition containing the present conjugate or a salt thereof (hereinafter also referred to as "the present composition") is provided.

The present composition can be used, for example, in various pharmaceutical applications, depending on the type of the aptamer to be contained. In the following, as an example of the application of the present composition, a case of use in pharmaceutical applications is described.

When the aptamer contained in the present composition is an anti-FGF2 aptamer, the present composition can be used, for example, as a therapeutic or prophylactic medicine for diseases accompanied by angiogenesis, bone/cartilage diseases, or pain. More preferably, the present composition can be used as a therapeutic or prophylactic medicine for diseases accompanied by angiogenesis such as age-related macular degeneration and the like, and bone or cartilage diseases such as osteoporosis, rheumatoid arthritis, osteoarthritis, bone fracture and the like, and pain.

When the aptamer contained in the present composition is an anti-autotaxin aptamer, the present composition can be used, for example, as a therapeutic or prophylactic medicine for diseases involving fibrotic change in an organ or tissue, particularly diseases accompanied by fibrosis in any of various tissues. More preferably, the present composition can be used as a therapeutic or prophylactic medicine for pulmonary fibrosis, prostatic hypertrophy, myocardial fibrotic change, myocardial fibrosis, musculoskeletal fibrosis, myelofibrosis, uterine fibroids, scleroderma, post-surgical adhesions, post-surgical scars, burn scars, hypertrophic scars, keloids, atopic dermatitis, peritoneal sclerosis, asthma, liver cirrhosis, chronic pancreatitis, scirrhous gastric cancer, liver fibrosis, renal fibrosis, fibrotic vascular disease, retinopathy due to fibrotic microangiopathy which is a complication of diabetes, neurosis, nephropathy, glomerulonephritis, tubulointerstitial nephritis, hereditary kidney disease, arteriosclerotic peripheral arteritis, and proliferative vitreoretinopathy.

The present composition may contain components other than the present conjugate or a salt thereof. Examples of such components include pharmaceutically acceptable carriers. As examples of the pharmaceutically acceptable carrier, excipients such as sucrose, starch, mannitol, sorbitol, lactose, glucose, cellulose, talc, calcium phosphate, and calcium carbonate; binders such as cellulose, methylcellulose, hydroxypropylcellulose, polypropylpyrrolidone, gelatin, gum arabic, polyethylene glycol, sucrose, and starch; disintegrants such as starch, carboxymethylcellulose, hydroxypropylstarch, sodium-glycol-starch, sodium hydrogen carbonate, calcium phosphate, and calcium citrate; lubricants such as magnesium stearate, Aerosil, talc, and sodium lauryl sulfate; flavoring agents such as citric acid, menthol, glycyrrhizin-ammonium salt, glycine, and orange powder; preservatives such as sodium benzoate, sodium hydrogen sulfite, methylparaben, and propylparaben; stabilizers such as citric acid, sodium citrate, and acetic acid; suspending agents such as methylcellulose, polyvinylpyrrolidone, and aluminum stearate; dispersing agents such as surfactants; diluents such as water, physiological saline, and orange juice; base waxes such as cacao butter, polyethylene glycol, and kerosene; and the like can be mentioned, but these are not limitative.

The administration route of the present composition is not particularly limited, and examples thereof include oral administration, parenteral administration, and the like. Preparations suitable for oral administration are a solution prepared by dissolving an effective amount of ligand in a diluent such as water, physiological saline, or orange juice; capsules, sachets or tablets comprising an effective amount of ligand in solid or granular form; a suspension prepared by suspending an effective amount of active ingredient in an appropriate dispersion medium; an emulsion prepared by dispersing and emulsifying a solution of an effective amount of active ingredient in an appropriate dispersion medium, and the like.

As preparations suitable for parenteral administration (e.g., intravenous administration, subcutaneous administration, intramuscular administration, topical administration, intraperitoneal administration, intranasal administration, pulmonary administration and the like), aqueous and non-aqueous isotonic sterile injectable liquids are available, which may comprise an antioxidant, a buffer, a bacteriostatic agent, an isotonizing agent and the like.

The dosage of the present composition varies depending on the kind and activity of active ingredient, seriousness of disease, animal species being the subject of administration, drug tolerability of the subject of administration, body weight, age and the like, and the usual dosage, based on the amount of active ingredient per day for an adult, can be about 0.0001 to about 100 mg/kg, for example, about 0.0001 to about 10 mg/kg, preferably about 0.005 to about 1 mg/kg.

### (4. Method for producing aptamer having extended blood half-life)

In one embodiment of the present invention, a method for producing an aptamer having an extended blood half-life, comprising a step of bonding polyethyloxazoline to an aptamer (hereinafter also referred to as "the present production method") is provided. In the present production method, an aptamer having an extended blood half-life can be produced by bonding polyethyloxazoline to an aptamer to form a conjugate of the aptamer and the polyethyloxazoline.

In the case of the branched polyethyloxazoline-added aptamer, it can be produced, for example, by adding a linker to the 5' end of the aptamer by a Michael addition reaction, and then adding a branched polyethyloxazoline by a dipolar cycloaddition reaction. More specifically, it can be produced, for example, by the method described in the Examples.

In the case of the linear polyethyloxazoline-added aptamer, it can be produced, for example, by bonding a linker to the 5' end of the aptamer by using a crosslinking reaction between an NHS ester and a primary amine, and then adding a linear polyethyloxazoline by a dipolar cycloaddition reaction. More specifically, it can be produced, for example, by the method described in the Examples.

### (5. Method for stabilizing aptamer)

In one embodiment of the present invention, a method for stabilizing an aptamer, comprising bonding polyethyloxazoline to an aptamer (hereinafter also referred to as "the present stabilizing method") is provided. In the present stabilizing method, the blood half-life can be extended by bonding polyethyloxazoline to an aptamer to form a conjugate of the aptamer and the polyethyloxazoline.

As a method for preparing the conjugate, for example, the method described in the above (4. Method for producing aptamer having extended blood half-life) can be used.

### (6. Others)

In one embodiment of the present invention, the following invention using a conjugate of an aptamer and polyethyloxazoline, or a salt thereof is provided.
(1) A therapeutic or prophylactic medicine for any disease, comprising a conjugate of an aptamer and polyethyloxazoline, or a salt thereof as an active ingredient.
(2) A method for treating or preventing any disease, comprising administering a therapeutically effective amount of a conjugate of an aptamer and polyethyloxazoline, or a salt thereof to a subject.
(3) Use of a conjugate of an aptamer and polyethyloxazoline, or a salt thereof in the manufacture of a medicine for treating or preventing any disease.
(4) A conjugate of an aptamer and polyethyloxazoline, or a salt thereof for use in the treatment or prevention of any disease.

In (1) to (4) of the present embodiment, the disease to be treated or prevented can be appropriately set depending on the type of the conjugate of an aptamer and polyethyloxazoline, or a salt thereof to be used. That is, the disease to be treated or prevented is not particularly limited as long as it is a disease that can be treated or prevented by the conjugate of an aptamer and polyethyloxazoline, or a salt thereof to be used.

In another embodiment of the present invention, the following invention is provided.
(1) A therapeutic or prophylactic medicine for a disease accompanied by angiogenesis, a bone/cartilage disease, or pain, comprising a conjugate of an anti-FGF2 aptamer and polyethyloxazoline, or a salt thereof as an active ingredient.
(2) A method for treating or preventing a disease accompanied by angiogenesis, a bone/cartilage disease, or pain, comprising administering a therapeutically effective amount of a conjugate of an anti-FGF2 aptamer and polyethyloxazoline, or a salt thereof to a subject.
(3) Use of a conjugate of an anti-FGF2 aptamer and polyethyloxazoline, or a salt thereof in the manufacture of a medicine for treating or preventing a disease accompanied by angiogenesis, a bone/cartilage disease, or pain.
(4) A conjugate of an anti-FGF2 aptamer and polyethyloxazoline, or a salt thereof for use in the treatment or prevention of a disease accompanied by angiogenesis, a bone/cartilage disease, or pain.

The disease accompanied by angiogenesis, bone/cartilage disease, or pain in the present embodiment may be, for example, one described in the above-mentioned (3. Composition).

In another embodiment of the present invention, the following invention is provided.
(1) A therapeutic or prophylactic medicine for a disease involving fibrotic change in an organ or tissue, particularly a disease accompanied by fibrosis in any of various tissues, comprising a conjugate of an anti-autotaxin aptamer and polyethyloxazoline, or a salt thereof as an active ingredient.
(2) A method for treating or preventing a disease involving fibrotic change in an organ or tissue, particularly a disease accompanied by fibrosis in any of various tissues, comprising administering a therapeutically effective amount of a conjugate of an anti-autotaxin aptamer and polyethyloxazoline, or a salt thereof to a subject.
(3) Use of a conjugate of an anti-autotaxin aptamer and polyethyloxazoline, or a salt thereof in the manufacture of a medicine for treating or preventing a disease involving fibrotic change in an organ or tissue, particularly a disease accompanied by fibrosis in any of various tissues.
(4) A conjugate of an anti-autotaxin aptamer and polyethyloxazoline, or a salt thereof for use in the treatment or prevention of a disease involving fibrotic change in an organ or tissue, particularly a disease accompanied by fibrosis in any of various tissues.

The disease involving fibrotic change in an organ or tissue in the present embodiment may be, for example, one described in the above-mentioned (3. Composition).

### [Example]

The present invention is described in more detail in the following using Examples, but this does not limit the scope of the present invention. The documents cited throughout the present specification are incorporated in their entirety into the present specification by reference.

### (1. Synthesis of branched PEOZ-, linear PEOZ-, and PEG-added aptamers)

For the branched PEOZ-added aptamer, a Mal-bis-PEG3-DBCO linker (CP-2070, manufactured by Conju-Probe) was added by a Michael addition reaction to a Thiol C6 linker attached to the 5' end of the aptamer. This linker has DBCO at each end of two branched PEG chains, and was synthesized by binding Poly(2-ethyl-2-oxazoline)-azide (HR12.0500/05.04A (molecular weight 50,000) or HR12.0250/05.04A (molecular weight 25,000), manufactured by AVROXA) to both DBCOs by a dipolar cycloaddition reaction. For the linear PEOZ-added aptamer, a DBCO-PEG4-NHS linker (CP-2028, manufactured by Conju-Probe) was bonded to an amino C6 linker attached to the 5' end of the aptamer by using a crosslinking reaction between an NHS ester and a primary amine, and further synthesized by adding Poly(2-ethyl-2-oxazoline)-azide (HR12.0500/05.04A, manufactured by AVROXA) by a dipolar cycloaddition reaction. The PEG-added aptamer was synthesized by adding a polyethylene glycol having a molecular weight of 40,000 (SUNBRIGHT GL2-400TS01, manufactured by NOF CORPORATION) to an amino C6 linker attached to the 5' end of the aptamer. In the following, 25 kDa PEOZ was used (partially) only in (4. Evaluation of blood retention of branched PEOZ-, linear PEOZ-, and PEG-added anti-FGF2 aptamers), and in other tests, 50 kDa PEOZ was used.

### (2. Evaluation of binding activity of branched PEOZ-added anti-FGF2 aptamer to FGF2)

Using the anti-FGF2 aptamer, the binding activity of the branched PEOZ-added aptamer to FGF2 was evaluated using a surface plasmon resonance method. BIAcore (trademark) T200 (manufactured by cytiva) was used for measurement. According to the protocol of cytiva, human FGF2 (AF-100-18B, manufactured by Peprotech) was immobilized at about 1000 RU on Series S Sensor Chip CM4 (29104989, manufactured by cytiva). For preparation of a running buffer and an aptamer solution for analyte, 300 mmol/l NaCl, 5.4 mmol/l KCl, 0.8 mmol/l MgCl₂, 1.8 mmol/l CaCl₂, 20 mmol/l Tris (pH 7.6), 0.05% Tween 20 were used. Aptamer solutions were prepared at 2 µg/mL and 125 µg/mL as free oligonucleic acid concentrations and injected. A 2 mol/l NaCl solution was used for regeneration of the sensor chip. A sensorgram obtained as a result of measurement is shown in Fig. 1. Similar to the PEG-added aptamer which is a positive control (Fig. 2), a concentration-dependent increase in signal was observed, and it was found that the branched PEOZ-added aptamer binds to FGF2.

### (3. Evaluation of in vitro inhibitory activity of branched PEOZ-added anti-FGF2 aptamer)

Using the anti-FGF2 aptamer, the cell stimulation inhibitory activities by FGF2 of the branched PEOZ-added aptamer and the PEG-added aptamer were compared. In NIH3T3 cells, when the signal transduction system is activated by cell stimulation by FGF2, the MAP kinase pathway, PIK3/AKT1 pathway, and the like are activated via FRS2, Grb2, and SOS, and the expression of various growth factors and receptor genes such as VEGF-A, VEGF-C, HGF, angiopoietin-2, VEGFR, and PDGFR-α is finally induced. At that time, it is known that ERK (Extracellular Signal-regulated Kinase) is phosphorylated. When NIH3T3 cells were stimulated with human FGF2 (AF-100-18B, manufactured by Peprotech) (1 nmol/ml), an aptamer was added into the medium, and phosphorylated ERK after 30 min was measured by an Enzyme-Linked Immunosorbent Assay (ELISA) method. For ELISA, ERK1 (phospho T202+Y204) + ERK2 (phospho T185+Y187) + total ERK ELISA Kit (ab176660, manufactured by Abcam) was used. By measuring absorbance at 450 nm using a microplate reader system (SpectraMax (registered trademark) 190, manufactured by Molecular Devices), the amount of phosphorylated ERK (p-ERK) and the amount of total ERK in samples were quantified. The phosphorylation level of ERK is expressed as a ratio of the absorbance of p-ERK to the absorbance of total ERK (p-ERK/total ERK ratio). The p-ERK/total ERK ratio (R0) when no aptamer was added to the medium was taken as 100%, and the phosphorylation inhibition rate of ERK by FGF2 stimulation was determined from the p-ERK/total ERK ratio (R) at each concentration by the following formula. R0 and R were values obtained by subtracting the p-ERK/total ERK ratio when no aptamer and FGF2 were added to the medium as a baseline. $Phosphorylation rate of ERK by FGF 2 = \left(R/R0\right) \times 100$ 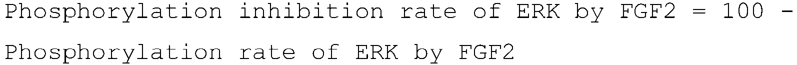 Phosphorylation inhibition rate of ERK by FGF2 = 100 - Phosphorylation rate of ERK by FGF2

The aptamer concentration required to inhibit ERK phosphorylation by FGF2 by 50% (IC50) was determined by non-linear regression using inhibition rate data. For analysis, GraphPad Prism7 (manufactured by Graphpad Software) was used. The inhibitory effect of ERK phosphorylation in cells after FGF2 stimulation by addition of the aptamer to which branched PEOZ or PEG was added is shown in Fig. 3. Addition of either aptamer showed concentration-dependent inhibitory activity, and the IC50 values (mean±standard deviation) of branched PEOZ and PEG were 112.5±25.9 and 127.1±22.5 pmol/l, respectively, which were comparable. From the above, it was shown that the branched PEOZ-added aptamer of the present invention has a functional inhibitory activity in living cells, comparable to that of the PEG-added aptamer.

### (4. Evaluation of blood retention of branched PEOZ-, linear PEOZ-, and PEG-added anti-FGF2 aptamers)

Using the anti-FGF2 aptamer, blood retention in mice of the branched PEOZ-, linear PEOZ-, and PEG-added aptamers was compared. Each aptamer was intravenously administered to 6- or 7-week-old male C57BL/6J mice (manufactured by Japan SLC, Inc.) at 0.5 mg/kg. At 1, 3, 6, 24, 48, and 72 hr after administration, mice were anesthetized with isoflurane (008803998, manufactured by Viatris), and about 500 µL of mouse blood was collected by cardiac blood sampling. The collected blood was placed in CAPIJECT II (EDTA·2K) (CJ-2DK, manufactured by Terumo Corporation), centrifuged at 2,300 x g for 10 min at 4°C with a micro high-speed refrigerated centrifuge (MX-301, manufactured by TOMY SEIKO CO., LTD.), and the obtained supernatant was recovered as plasma. The aptamer concentration in the obtained plasma was measured using an Enzyme-Linked oligonucleotide sorbent assay (ELOSA) method. The method is shown below. As detection probes, an oligonucleic acid whose 5'-end was labeled with Fluorescein (FAM) and an oligonucleic acid whose 3'-end was labeled with an amino group were used. A plasma sample diluted with PBS containing 1 w/v% SDS and the FAM-labeled detection probe were mixed, allowed to stand at 80°C under light-shielded conditions for 10 min, and then rapidly cooled on ice to hybridize the FAM-labeled detection probe and the aptamer. This was used as a pretreatment solution. The amino group-labeled detection probe was immobilized on a 96-well (round-bottom) plate coated with active ester (manufactured by Sumitomo Bakelite Co., Ltd.), the pretreatment solution was added thereto, and the plate was shaken at 60°C under light-shielded conditions for 1 hr, and then rapidly cooled on ice to hybridize the amino group-labeled detection probe and the aptamer. The plate was washed with a washing solution (PBS containing 0.05 v/v% Tween 20), treated with 100 µL of a solution obtained by diluting a stock solution of horseradish peroxidase (HRP)-labeled anti-FITC antibody (6400-05, manufactured by SouthernBiotech) 8000 times with PBS, and the antibody was bound to FAM. Thereafter, color was developed using a color development kit T for peroxidase (ML-1120T, manufactured by Sumitomo Bakelite Co., Ltd.), and absorbance at 450 nm was measured using a microplate reader system (SpectraMax (registered trademark) M2, SpectraMax (registered trademark) 190, manufactured by Molecular Devices).

The blood concentration transition of each aptamer is shown in Fig. 4. The blood half-lives of the branched 25 kDax2, branched 50 kDax2, linear 50 kDa PEOZ-added aptamers and the PEG-added aptamer were 5.55, 18.05, 6.35 hr, and 7.70 hr, respectively. From this, the blood retention of the branched 50 kDax2 PEOZ-added aptamer was equal to or superior to that of the PEG-added aptamer. In addition, the branched 25 kDax2 and 50 kDax2 PEOZ-added aptamers showed blood retention equal to or superior to that of the linear 50 kDa, and all of them drastically increased blood retention compared with the aptamer alone (without addition).

### (5. Evaluation of enzyme inhibitory activity of PEOZ-added anti-autotaxin aptamer)

Using the anti-autotaxin aptamer, the enzyme inhibitory activities on autotaxin of the branched PEOZ-added aptamer and the PEG-added aptamer were compared. As the anti-autotaxin aptamer, an aptamer consisting of the nucleotide sequence represented by the formula (1), which is a modified variant of the aptamer having the nucleotide sequence represented by SEQ ID NO: 1, was used.
The formula (1): 5'-tC(M)U(M)G(M)A(M)G(M)G(M)gA(M)AsA(M)C(F)A(M)ggU(F)U(F)U(F)U(F)G (M)C(F)U(M)cC(M)U(M)cG(M)G(M)A(M)_idT-3'
(uppercase letters indicate RNA, lowercase letters indicate DNA, parentheses in nucleotides indicate modification at the 2' position of ribose, F indicates a fluorine atom, and M indicates an O-methyl group; idT at the sequence end indicates modification by inverted-dT, and s in the sequence indicates that the phosphate group linking nucleotides to each other has been phosphorothioated.)

The enzyme inhibitory activity was evaluated by the following method. As a substrate for autotaxin, a phosphodiester bond-containing synthetic substrate p-nitrophenyl thymidine 5'-monophosphate (p NP-TMP) (T4510, manufactured by SIGMA) was selected (hereinafter referred to as NPP2 inhibition assay). The phosphodiester bond is cleaved by hydrolysis, and p-nitrophenol is liberated. This p-nitrophenol develops a yellow color, which is detected. For the assay, a 96-well plate (UV-Star 96-well microplate flat-bottom, 655801, Greiner BIO-ONE) was used, and it was performed with a reaction solution volume of 100 µL. Solution A was used as a reaction solution. Here, solution A is a mixed solution of 145 mmol/l NaCl, 5.4 mmol/l KCl, 0.8 mmol/l MgCl₂, 1.8 mmol/l CaCl₂, 20 mmol/l Tris (pH 7.6), 0.05% Tween 20. To 50 µL of an aptamer solution prepared to each concentration with solution A, 10 µL of pNP-TMP prepared to 10 mmol/l with solution A similarly was added and mixed well, and then heated at 37°C for 5 min. On the other hand, 40 µL of 6 ng of autotaxin diluted with solution A was prepared and heated at 37°C for 5 min. After heating, both were mixed to start an enzyme reaction. The final autotaxin concentration in the reaction solution is 0.3 nM, and the final substrate concentration is 1 mM. The plate containing the reaction solution was heated at 37°C for 6 hr, then set in a microplate reader system SpectraMax (registered trademark) M2 (manufactured by Molecular Devices), and absorbance was determined at a wavelength of 405 nm. The absorbance when no aptamer was contained (A0) was taken as 100%, and the enzyme activity inhibition rate was determined from the absorbance (A) at each concentration by the following formula. A0 and A were values obtained by subtracting the value immediately after the start of the enzyme reaction (0 hr) as a background. $Enzyme activity rate = \left(A/A0\right) \times 100$ $Enzyme activity inhibition rate = 100 - enzyme activity rate$

The aptamer concentration required to inhibit enzyme activity by 50% (IC₅₀) was determined by non-linear regression using inhibition rate data. For analysis, GraphPad Prism7 (manufactured by Graphpad Software) was used. The enzyme activity inhibitory effect by the branched PEOZ- and PEG-added aptamers is shown in Fig. 5. Either adduct showed concentration-dependent inhibitory activity, and the IC₅₀ values (mean±standard deviation) of branched PEOZ and PEG were 943.1±105.0 and 634.2±31.5 pmol/L, respectively, and were comparable. From the above, it was shown that the branched PEOZ-added anti-autotaxin aptamer of the present invention has an enzyme inhibitory activity comparable to that of the PEG-added anti-autotaxin aptamer.

### (6. Evaluation of blood retention of branched PEOZ- and PEG-added anti-autotaxin aptamers)

Using the anti-autotaxin aptamer, blood retention in mice of the branched PEOZ- and PEG-added aptamers was compared. Each aptamer was intravenously administered to 6-week-old male C57BL/6J mice (manufactured by Japan SLC, Inc.) at 0.5 mg/kg. At 1, 3, 6, 24, 48, and 72 hr after administration, mice were anesthetized with isoflurane (008803998, manufactured by Viatris), and about 500 µL of mouse blood was collected by cardiac blood sampling. The collected blood was placed in CAPIJECT II (EDTA·2K) (CJ-2DK, manufactured by Terumo Corporation), centrifuged at 2,300 x g for 10 min at 4°C with a micro high-speed refrigerated centrifuge (MX-301, manufactured by TOMY SEIKO CO., LTD.), and the obtained supernatant was recovered as plasma.

The aptamer concentration in the obtained plasma was measured using an Enzyme-Linked oligonucleotide sorbent assay (ELOSA) method. The method is shown below. As a detection probe, an oligonucleic acid of a sequence whose 5'-end was labeled with Fluorescein (FAM): 5'-U(M)C(M)C(M)G(M)A(M)G(M)G(M)A(M)G(M)C(M)A(M)A(M)A(M)A(M)C(M)-3' (which is a modification of SEQ ID NO: 2 (1): 5'-UCCGAGGAGCAAAAC-3' (SEQ ID NO: 2), parentheses in nucleotides indicate modification at the 2'-position of ribose, and M indicates an O-methyl group), and an oligonucleic acid of a sequence whose 3'-end was labeled with biotin: 5'-C(M)U(M)G(M)U(M)U(M)U(M)C(M)C(M)C(M)U(M)C(M)A(M)G(M)A(M)-3' (which is a modification of SEQ ID NO: 3 (1): 5'-CUGUUUCCCUCAGA-3' (SEQ ID NO: 3), parentheses in nucleotides indicate modification at the 2'-position of ribose, and M indicates an O-methyl group) were used. A plasma sample diluted with 1 w/v% skim milk and the FAM-labeled detection probe were mixed, allowed to stand at 80°C under light-shielded conditions for 10 min, and then rapidly cooled on ice to hybridize the FAM-labeled detection probe and the aptamer. This was used as a pretreatment solution. After the biotin-labeled detection probe was immobilized on a 96-well plate coated with streptavidin (IMMOBILIZER STREPTAVIDIN F8 clear (436020, manufactured by Thermo Fisher Scientific)), blocking was performed with 1 w/v% skim milk. The pretreatment solution was added thereto, and the plate was shaken at 50°C under light-shielded conditions for 1 hr, and then rapidly cooled on ice to hybridize the biotin-labeled detection probe and the aptamer. The plate was washed with a washing solution (PBS containing 0.05 v/v% Tween 20), treated with 100 µL of a solution obtained by diluting a stock solution of horseradish peroxidase (HRP)-labeled anti-FITC antibody (6400-05, manufactured by SouthernBiotech) 8000 times with PBS, and the antibody was bound to FAM. Thereafter, color was developed using a color development kit T for peroxidase (ML-1120T, manufactured by Sumitomo Bakelite Co., Ltd.), and absorbance at 450 nm was measured using a microplate reader system (SpectraMax (registered trademark) M2, manufactured by Molecular Devices).

The blood concentration transition of each aptamer is shown in Fig. 6. The blood half-lives of the branched PEOZ-added and PEG-added aptamers were 6.30 and 3.75 hr, respectively. From this, it was found that the blood retention of the branched PEOZ-added aptamer is superior to that of the PEG-added aptamer.

### [Industrial Applicability]

The present invention can extend the blood half-life of an aptamer, and therefore is useful in various medical applications using an aptamer. This application is based on a patent application No. 2024-012285 filed in Japan (filing date: January 30, 2024), the contents of which are incorporated in full herein.

## Claims

1. A conjugate of an aptamer and polyethyloxazoline, or a salt thereof.

2. The conjugate or a salt thereof according to claim 1, wherein the polyethyloxazoline forms a branched chain.

3. The conjugate or a salt thereof according to claim 1 or 2, wherein the polyethyloxazoline is bonded to the 5' end of the aptamer.

4. The conjugate or a salt thereof according to claim 1 or 2, wherein the polyethyloxazoline is two in number and the two polyethyloxazolines form a branched chain via a linker.

5. The conjugate or a salt thereof according to claim 2, wherein each molecular weight of the branched chains of the polyethyloxazoline is 20 to 60 kDa.

6. The conjugate or a salt thereof according to claim 4, wherein the polyethyloxazoline is bonded to the linker by a dipolar cycloaddition reaction.

7. The conjugate or a salt thereof according to claim 6, wherein the dipolar cycloaddition reaction is a dipolar cycloaddition reaction between an azide on the polyethyloxazoline side and a dibenzocyclooctyne on the linker side.

8. The conjugate or a salt thereof according to claim 1, wherein the polyethyloxazoline is bonded to the 5' end of the aptamer via a linker.

9. The conjugate or a salt thereof according to claim 8, wherein the structure of the polyethyloxazoline-linker moiety is the following: 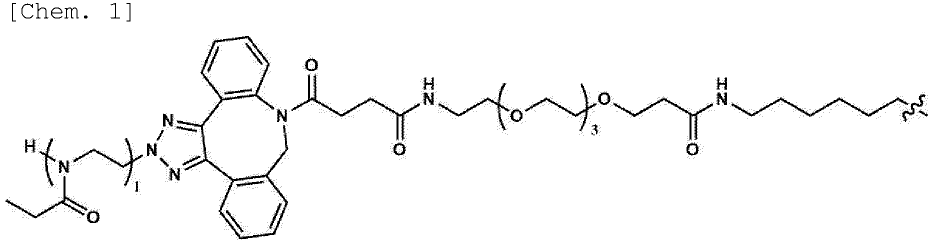 wherein l indicates a degree of polymerization, and l is 200 to 600.

10. The conjugate or a salt thereof according to claim 4, wherein the structure of the polyethyloxazoline-linker moiety is the following: 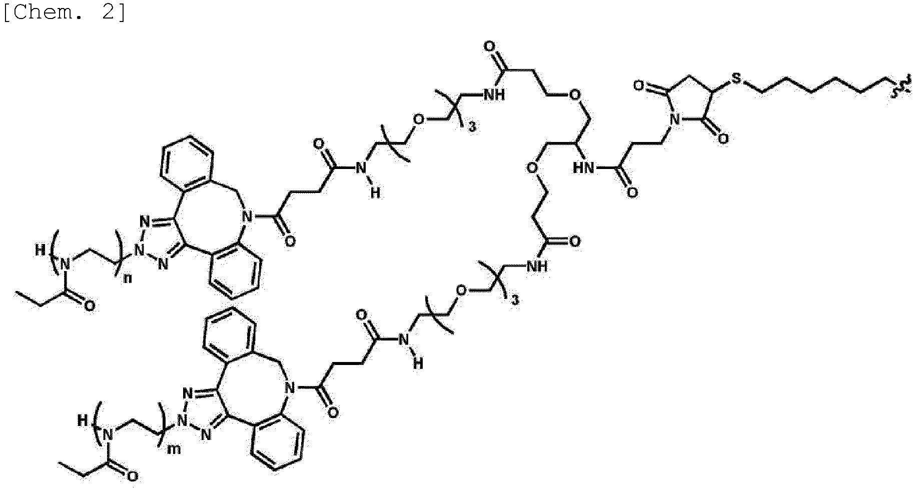 wherein m and n each indicate a degree of polymerization, m is 200 to 600, and n is 200 to 600.

11. The conjugate or a salt thereof according to claim 1 or 2, wherein the aptamer is an anti-FGF2 aptamer or an anti-autotaxin aptamer.

12. The conjugate or a salt thereof according to claim 1 or 2, wherein the aptamer is an aptamer consisting of a nucleotide sequence represented by the following formula (1):
tC(M)U(M)G(M)A(M)G(M)G(M)gA(M)AsA(M)C(F)A(M)ggU(F)U(F)U(F)U(F)G (M)C(F)U(M)cC(M)U(M)cG(M)G(M)A(M)_idT (1)
(uppercase letters indicate RNA, lowercase letters indicate DNA, parentheses in nucleotides indicate modification at the 2' position of ribose, F indicates a fluorine atom, and M indicates an O-methyl group; idT at the sequence end indicates modification by inverted-dT, and s in the sequence indicates that the phosphate group linking nucleotides to each other has been phosphorothioated.)

13. A composition comprising the conjugate or a salt thereof according to claim 1 or 2.

14. A method for producing an aptamer having an extended blood half-life, comprising a step of bonding polyethyloxazoline to an aptamer.

15. The production method according to claim 14, wherein the polyethyloxazoline forms a branched chain.

16. The production method according to claim 14 or 15,wherein the polyethyloxazoline is bonded to the 5' end of the aptamer.

17. The production method according to claim 14 or 15,
wherein the polyethyloxazoline branched via a linker is bonded to the aptamer.

18. The production method according to claim 15, wherein the polyethyloxazoline in which each molecular weight of the branched chains is 20 to 60 kDa is bonded to the aptamer.

19. The production method according to claim 17, wherein the polyethyloxazoline and the linker are bonded by a dipolar cycloaddition reaction.

20. The production method according to claim 19, wherein the polyethyloxazoline and the linker are bonded by a dipolar cycloaddition reaction between an azide on the polyethyloxazoline side and a dibenzocyclooctyne on the linker side.

21. The production method according to claim 14 or 15, wherein the polyethyloxazoline branched via a linker is bonded to the aptamer by a Michael addition reaction between a maleimide group on the linker side and a thiol group on the aptamer side.

22. The production method according to claim 14, wherein the polyethyloxazoline is bonded to the 5' end of the aptamer via a linker.

23. The production method according to claim 22, wherein the structure of the polyethyloxazoline-linker moiety is the following: 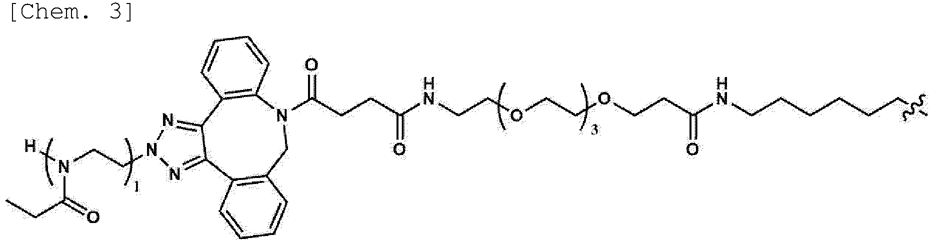 wherein l indicates a degree of polymerization, and l is 200 to 600.

24. The production method according to claim 17, wherein the structure of the polyethyloxazoline-linker moiety is of the following structural formula: 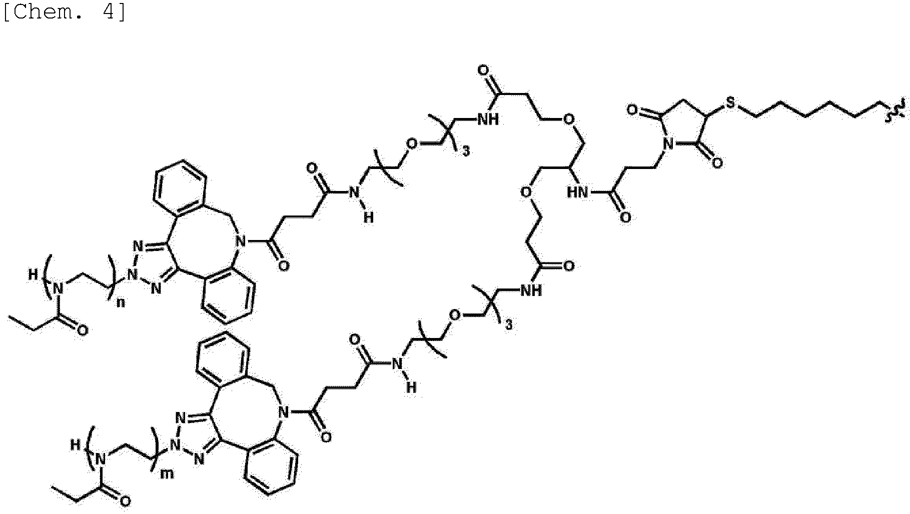 wherein m and n each indicate a degree of polymerization, m is 200 to 600, and n is 200 to 600.

25. A method for stabilizing an aptamer, comprising bonding polyethyloxazoline to an aptamer.
